# EUROPEAN PATENT APPLICATION

(11) **EP 4 617 661 A1**
(43) Date of publication of application: **17.09.2025**
(21) Application number: 24163417.9
(22) Date of filing: 14.03.2024
(51) Int. Cl.: G01N 33/50, C12N 15/10

(54) **CELL-FREE EXPRESSION AND SCREENING MICROFLUIDIC PLATFORM FOR PEPTIDE DRUG DISCOVERY**

(71) Applicant: ETH Zurich, 8092 Zürich (CH); Universität Zürich, 8006 Zürich (CH)
(72) Inventor: Chen, Junyue, 8046 Zürich (CH); Cola, Reto Bruna, 8600 Dübendorf (CH); Demello, Andrew James, 8044 Zürich (CH); Patriarchi, Tommaso, 8044 Zürich (CH); Stavrakis, Stavros, 8044 Zürich (CH)
(74) Representative: Schmitz, Joseph

(57) **Abstract**

A process for screening a plasmid library encoding for peptides for one or more peptides that functionally activate a G protein-coupled receptor in a cell, by contacting the peptides with a cell expressing a G protein-coupled receptor, which functions as a sensor, and isolating the cells that functionally activate a G protein-coupled receptor to determine the sequence encoding the peptides.

## Description

### TECHNICAL FIELD

The present invention relates to a process for screening a plasmid library encoding for peptides that functionally activate a G protein-coupled receptor in a cell, as well as an apparatus configured to carry out said process.

### PRIOR ART

Cell-free Directed Evolution of a Protease in Microdroplets at Ultrahigh Throughput, J Holstein, C Gylstorff, and F Hollfelder, ACS Synth. Biol. 2021, 10, 252-257 discloses a three-step workflow separating three previously incompatible steps that thus far could not be carried out at once. In a first step, plasmids were amplified by rolling circle amplification in a microfluidic water-in-oil droplet. In a subsequent step, reagents for *in vitro* protein expression were injected by picoinjection into the droplet to express protease variants and then, in a final step, a substrate for the protease was also added via picoinjection. If hydrolysed, the fluorogenic casein substrate, emitted a fluorescent signal that was used to sort the droplets and determine the sequence of the active proteases. However, the three-step workflow is complicated and necessitates precise control, especially during the picoinjection steps to ensure the delivery of the right amount of polymerase and protein expression system.

A completely *in vitro* ultrahigh-throughput droplet-based microfluidic screening system for protein engineering and directed evolution A Fallah-Araghi, J Baret, M Ryckelynck and A Griffiths Lab Chip, 2012, 12, 882 discloses a process in which single genes encoding for a beta-galactosidase are compartmentalized in aqueous droplets, dispersed in inert carrier oil, and amplified using the polymerase chain reaction (PCR). After amplification, the droplets are fused one-to-one with droplets containing a cell-free coupled transcription-translation (IVTT) system and a substrate for a fluorogenic assay, i.e. fluorescein-di-beta-D-galactopyranoside. Fluorescence-activated electrocoalescence with an aqueous stream was then used to selectively recover genes from droplets containing the active variant of beta-galactosidase.

The drug development effort is a process requiring a significant upfront capital investment with no guarantee of success. Accordingly, to stay competitive, the pharmaceutical industry is under pressure to discover new active pharmaceutical ingredients (APIs) while bringing down the overall cost of drug development. With the advent of genomics and combinatorial chemistry and the adoption of high-throughput screening (HTS) methodologies, there has been a surge of data on potential therapeutic drug targets and libraries of molecules to be screened. For example, testing of large chemical libraries for a specific biological activity is often a lengthy and laborious undertaking. This has necessitated the development of even more cost-effective methodologies to efficiently screen a vast number of compounds against a variety of therapeutic targets.

The screening of compounds for activity against specific therapeutic targets has traditionally been done manually with typical throughputs of around 1000 assays per day. Nowadays, high-throughput drug screening assays are carried out in microtiter well plates, where the drug solutions are added to either target cells or target molecules. Using robotics, a reduction in reagents and materials can be achieved by increasing the number of wells per microtitre plate and accordingly the volume of the microtiter plate.

Using droplet-based microfluidics offers a drastic increase in the number of screened units since volumes drop to the picoliter scale and offers a massive reduction in sample consumption. Performing drug screens in droplets seems like a great solution, since they enable high-throughput screening of multiple drugs in a single experiment, resulting in the quick discovery of interesting therapeutic candidates. Despite the great potential of droplet-based microfluidics to enable cheaper and faster screens of drugs, microfluidics is lagging behind its promise. This discrepancy is due to several limitations that arise when translating drug screens into droplet-based microfluidics. Different candidate compounds must be loaded into the microfluidic device, a process which is very slow (minutes or hours) compared to droplet production *per se* (thousands per second) and this limits the throughput. When the number of chemically diverse drugs is on the scale of thousands, or millions, it becomes impossible to load all these compounds into different droplets. To achieve a continuous and automated production of droplets containing different candidate compounds, such as peptides or proteins, it has been proposed to include individual plasmids from a plasmid library in individual microfluidic water-in-oil droplets, where they can subsequently be amplified by rolling circle amplification via separate picoinjection of an *in vitro* transcription and translation (IVTT) system. These strategies, however, do not offer the possibility of being able to monitor the interaction between the candidate peptide compounds and their targets.

### SUMMARY OF THE INVENTION

It is therefore an objective of the present invention to provide a solution to the above-mentioned problem, i.e. to provide a method in which a large number of candidate compounds, which are encoded in a plasmid library, can be tested *in vivo* for their agonist or antagonist effect on a target, in the sense that a living cell is contacted with candidate compound.

In a first object, the present invention thus provides a process for screening a plasmid library encoding for peptides for one or more peptides that functionally activate a G protein-coupled receptor in a cell, comprising the step of:
- providing a first set of water-in-oil droplets comprising each a single plasmid encoding a candidate peptide or a pro-peptide thereof, a polymerase capable of rolling circle replication of plasmids, and an *in vitro* transcription/translation system, and optionally a protease,
- replicating the plasmids using the polymerase capable of rolling circle replication of plasmids and concomitantly expressing the candidate peptides or pro-peptides encoded thereon using the *in vitro* transcription/translation system, within the water-in-oil droplets, to form droplets comprising each a candidate peptide, wherein if a pro-peptide is expressed, the pro-peptide is hydrolysed to form droplets comprising each a candidate peptide,
- providing a second set of water-in-oil droplets, where each droplet comprises a one or more cells having a G protein-coupled receptor expressed on its surface, where said G protein-coupled receptor has a reporter moiety coupled to it and said reporter moiety is capable of emitting a signal, preferably an electromagnetic radiation signal, upon functional activation of the G protein-coupled receptor by a suitable candidate peptide,

- merging the droplets comprising a candidate peptide or a pro-peptide from the first set with the droplets comprising a one or more cells having a G
- protein-coupled receptor expressed on its surface from the second set, in a one-to-one fashion, to form a third set of combined droplets,
- in the third set of combined droplets, separating the combined droplets that emit a signal above a predetermined signal intensity threshold, to form a fourth set of combined droplets, from the combined droplets that emit a signal below a predetermined signal intensity threshold,
- in the fourth set of combined droplets, determining the sequences encoding the peptides or pro-peptides on the plasmids.

In a second object, the present invention further provides a microfluidic device configured to carry out the process according to the first object, the device comprising
- a microfluidic formation chamber for forming a first set of water-in-oil droplets, each comprising a single plasmid encoding a candidate peptide or a pro-peptide thereof, a polymerase capable of rolling circle replication of plasmids, and an in vitro transcription/translation system, and optionally a protease,
   wherein the formation chamber is fluidly connected to a reservoir of oil and a reservoir of an aqueous liquid comprising plasmids of a plasmid library, each plasmid comprising a sequence encoding a candidate peptide or a pro-peptide thereof, and a polymerase capable of rolling circle replication of plasmids, and an in vitro transcription/translation system, and optionally a protease,
- a microfluidic merging chamber fluidly connected to the formation chamber, for receiving the water-in-oil droplets from the formation chamber, for merging droplets of the first set comprising a candidate peptide with droplets of a second set, said droplets comprising a one or more cells having a G protein-coupled receptor expressed on its surface, in a one-to-one fashion, to form a third set of combined droplets,
   wherein the merging chamber preferably comprises electrodes capable of generating an electrical field across the merging chamber,
- a sorting chamber fluidly connected to the merging chamber, for receiving the combined droplets and separating the combined droplets that emit a signal above a predetermined signal intensity threshold, to form a fourth set of droplets, from the combined droplets that emit a signal below a predetermined signal intensity threshold.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

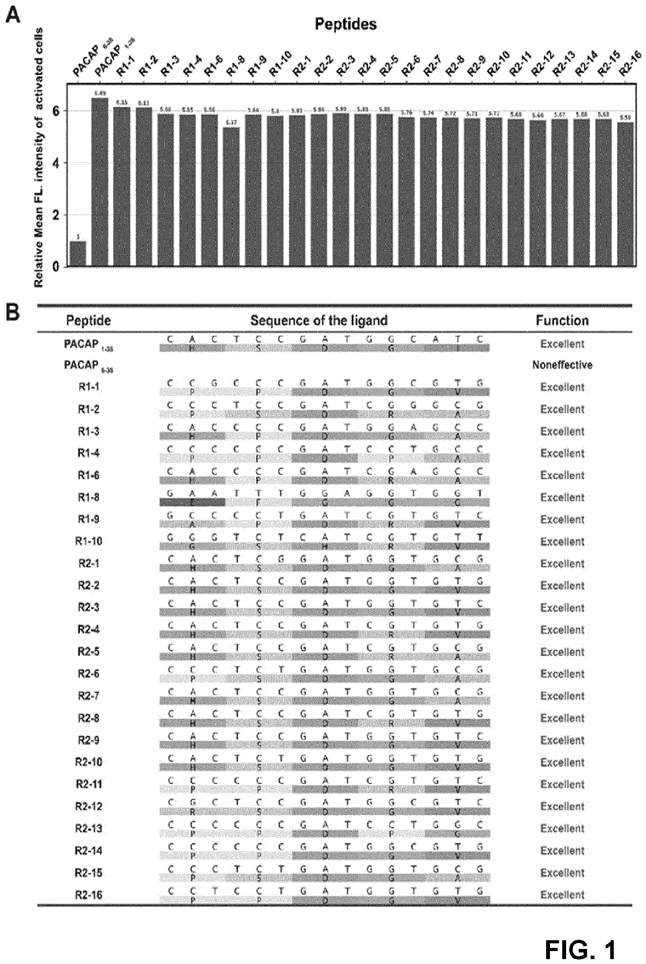

- Fig. 1: shows in 1A the relative mean fluorescence intensity of cells expressing PACLight1, normalized with respect to the fluorescence intensity of the PACAP ₆₋₃₈ antagonist, after being merged with droplets comprising the respective expression product of 100 fg/µL plasmid and shows in 1B the sequence information of each of the 25 penta-peptide from the library that lead to a functional activation of PCLight1.

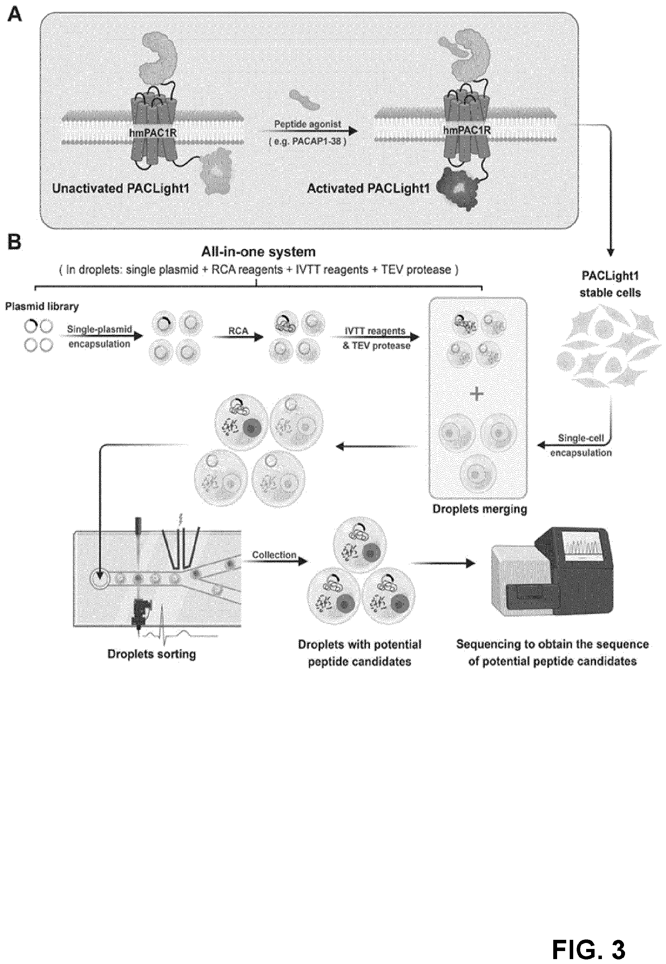

- Fig. 3: shows an example of a screening workflow according to the present invention, in which in 3A, the conformational change of the PACLight1 G-protein coupled receptor, upon binding the peptide agonist PACAP₁₋₃₈, is depicted an in which 3B, the steps of the screening method of: providing droplets comprising the single plasmids, IVTT reagent, RCA polymerase, TEV protease, replicating and expressing the protein encoded on the plasmids, merging with cells comprising a cell expressing the G-protein coupled receptor bearing a fluorescent moiety, and sorting the combined droplets that contain a peptide that functionally activates the receptor, and finally sequencing the plasmids to ascertain the sequence encoding the activating peptide, are depicted.

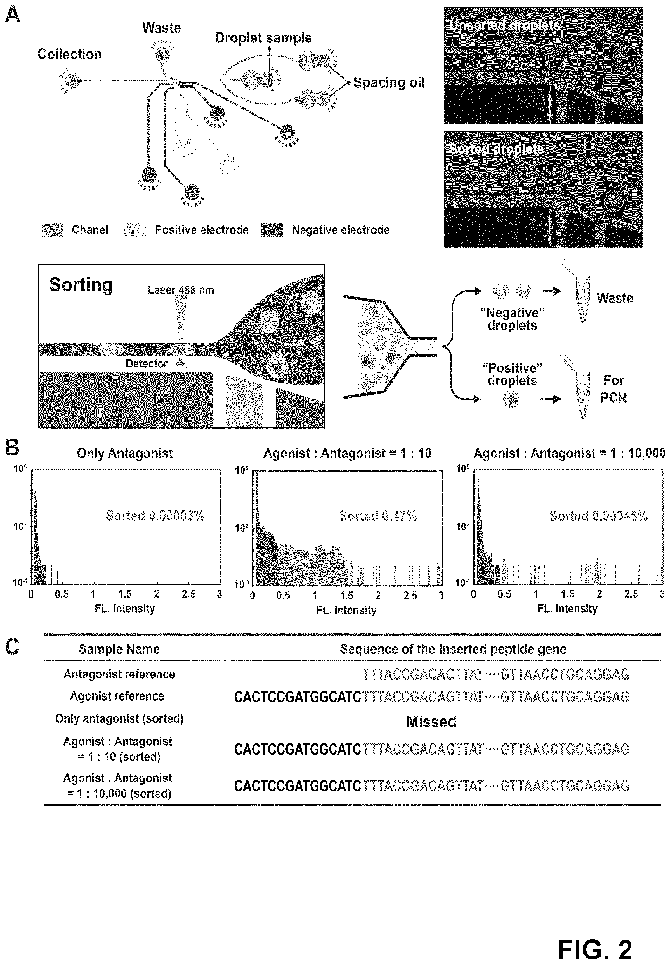

- Fig. 2: shows in 2A, on the top left, a schematic of the apparatus that may be used in the process for screening a plasmid library encoding for peptides that functionally activate a G protein-coupled receptor in a cell, where the combined droplets flow through a fluorescence-activated droplet sorting (FADS) chamber to be separated based on whether they emit a signal above or below a predetermined signal intensity. The sorting chamber is shown enlarged in the bottom left of Figure 2A depicting a fluorescence detection device equipped with a laser operating at 488 nm coupled to a control circuit that allows to deflect the combined droplets depending in the measured fluorescence via a positive electrode placed immediately before the bifurcation in a microfluidic Y-junction. On the top right, two microscope images of the sorting chamber are shown in the case where a droplet is discarded ("unsorted droplets", top) an in the case a droplet is retained for further analysis ("sorted droplet", bottom) of the plasmid, such as sequencing. In 2B, fluorescence intensity is plotted against droplet counts through a fluorescence-activated droplet sorting (FADS) chamber for three experiments, in which only combined droplets comprising plasmids encoding for the antagonist of a G protein-coupled receptor and one or more cells having said G protein-coupled receptor with a fluorescent reporter moiety, in which combined droplets comprising plasmids encoding for the antagonist of a G protein-coupled receptor and one or more cells having said G protein-coupled receptor with a fluorescent reporter moiety and combined droplets comprising plasmids encoding for the agonist of a G protein-coupled receptor and one or more cells having said G protein-coupled receptor with a fluorescent reporter moiety were introduced at a 10:1 ratio, and in which only combined droplets comprising plasmids encoding for the antagonist of a G protein-coupled receptor and one or more cells having said G protein-coupled receptor with a fluorescent reporter moiety, in which combined droplets comprising plasmids encoding for the antagonist of a G protein-coupled receptor and one or more cells having said G protein-coupled receptor with a fluorescent reporter moiety and combined droplets comprising plasmids encoding for the agonist of a G protein-coupled receptor and one or more cells having said G protein-coupled receptor with a fluorescent reporter moiety were introduced at a 10 000:1 ratio. As can be seen, when only plasmids encoding for the antagonist were used, virtually no positive (above fl. Intensity 0.4) combined droplets were detected, whereas in the case where a mix of plasmids encoding for both agonist and antagonist in different ratios were used, the combined droplets comprising the plasmids encoding for the agonist were recognized (fl. Intensity of more than 0.4) because of expressed peptide of the plasmid activated the G protein-coupled receptor with a fluorescent reporter moiety. Note that even at very dilute ratios of 10 000:1, the relatively few positive combined cells can still reliably be detected, and therefore can also be isolated to retrieve the relevant sequences encoded on the plasmid, as shown in Fig. 2C.

### DESCRIPTION OF PREFERRED EMBODIMENTS

In a first object, the present invention thus provides a process for screening a plasmid library encoding for peptides that functionally activate a G protein-coupled receptor in a cell.

In the context of the present invention, a plasmid library is a genomic library in which the polynucleotide fragments of interest are inserted into a plasmid vector such that the polynucleotide fragments of interest, which encode a peptide, may be expressed when the plasmid vector is in the presence of system capable of transcription and translation. Plasmid vectors are well-known and are commercially available, as are methods to generate genomic libraries from them. For example, a suitable plasmid that may be useful in the present invention is the pBEST plasmid.

In the context of the present invention, a peptide is a chain of amino acid residues linked by peptide bonds. It is noted that in general, the number of amino acid residues in a peptide is not limited in the context of the present invention, but will in general be from 4 to 600, and preferably 10 to 200. As an example, the peptide may comprise between four to hundreds of amino acid residues. The present invention is suitable for peptides that are post-translationally modified or that are not post-translationally modified. In the case a post-translational modification such as for example enzymatic cleavage or glycosylation is intended, the corresponding reactants such as a protease or a (glycosyl)transferase can be added in the aqueous liquid from which the droplets are formed.

G protein-coupled receptors (GPCRs), also known as seven-(pass)-transmembrane domain receptors, 7TM receptors, heptahelical receptors, serpentine receptors, and G protein-linked receptors (GPLR), form a large group of evolutionarily related proteins that are cell surface receptors that detect molecules outside the cell and activate cellular responses. They pass through the cell membrane seven times in form of six loops (three extracellular loops interacting with ligand molecules, three intracellular loops interacting with G proteins, an N-terminal extracellular region and a C-terminal intracellular region of amino acid residues, which is why they are sometimes referred to as seven-transmembrane receptors. Their ligands can bind either to the extracellular N-terminus and loops (e.g. glutamate receptors) or to the binding site within transmembrane helices (rhodopsin-like family), and they are generally activated by ligands (i.e. agonists).

The process according to the present invention comprises the step of:
- providing a first set of water-in-oil droplets comprising each a single plasmid encoding a candidate peptide or a pro-peptide thereof, a polymerase capable of rolling circle replication of plasmids, and an in vitro transcription/translation system. Stated alternatively, the content of the droplet comprises all elements for replication, translation of the gene encoding the candidate peptide and eventually hydrolysis of a peptide, as in what is commonly referred to as a "one-pot reaction".

In order to form and provide a first set of water-in-oil droplets comprising a single plasmid of the plasmid library, said single plasmid comprising a sequence encoding a candidate peptide, and a polymerase capable of rolling circle replication of plasmids, and an *in vitro* transcription/translation system, all the aforementioned components are included in an aqueous liquid, such as for example an aqueous buffer, so that when the water-in-oil droplets are formed from the aqueous liquid, a majority of the droplets that comprise plasmids are droplets that comprise a single plasmid of the plasmid library, and a sufficient amount of polymerase and *in vitro* transcription/translation system for expression of the peptide encoded in the plasmid. In order to ensure that the majority of droplets that are formed comprise a single plasmid of the plasmid library, or no more than a single plasmid of the plasmid library, the concentration of plasmid in the aqueous liquid from which the droplets are formed must be chosen within a suitable range. It has been found that a concentration of plasmid in the aqueous liquid of from 10 to 100 fg/pl leads to a high yield of droplets comprising one plasmid. The inclusion of plasmids in the droplets is governed by the Poisson distribution, and thus, it should be understood that the term "a majority of the droplets that comprise plasmids are droplets that comprise a single plasmid of the plasmid library " means that at least 65%, preferably at least 75%, and more preferably at least 80% of the droplets are droplets that comprise a single plasmid of the plasmid library, with the remainder of the droplets comprising two or more plasmids of the plasmid library.

During the formation of the droplets, which is preferably carried out in a microfluidic device, a Poisson distribution is achieved, where about 10% of the droplets comprise one plasmid and where about 2% of the droplets comprise two or more plasmids, and where about 88% of the droplets comprise no plasmids. It is therefore a preferred embodiment to form a first set of water-in-oil droplets, where the ratio of droplets comprising a single plasmid of the plasmid library to droplets comprising two or more plasmids of the plasmid library is higher than 3, preferably higher than 4 or more preferably higher than 5.

In a preferred embodiment, the first set of water-in-oil droplets are formed in a microfluidic device.

In a preferred embodiment, the first set of water-in-oil droplets formed in a microfluidic device may be pre-sorted to exclude droplets that comprise no plasmid by dyeing the plasmid DNA inside the droplets with a dye such as SYBR dye. Droplets that comprise no plasmid do not retain the dye and can be discarded, leaving droplets that comprise either one plasmid or two or more plasmids. As more plasmid DNA will mean more dyeing, via SYBR, for example, the pre-sorting may preferably be carried out after the step of replicating the plasmids with the polymerase capable of rolling circle replication of plasmids instead of immediately after the formation of the droplets.

The concentration of the polymerase capable of rolling circle replication of plasmids, and of the *in vitro* transcription/translation system must be chosen so that expression of the peptide encoded in the plasmid is ensured. It has been found that when the concentration of polymerase in the aqueous liquid is at least 0.5 U/µl or is at least 1 U/µl, and when the concentration of *in vitro* transcription/translation system is at least 20% v/v, preferably at least 25% v/v, based on the total volume of the aqueous liquid, expression of the peptide or pro-peptide is ensured.

In order to form a first set of water-in-oil droplets in a microfluidic device, the oil may be chosen from any suitable synthetic oil, for example a fluorinated oil, such as HFE-7500 available commercially from 3M and optionally, a surfactant such as a fluoro-surfactant CAS 297730-93-9.

A microfluidic device suitable for forming a first set of water-in-oil droplets may be a microfluidic flow-focusing device (20 µm height and 20 µm width at the flow-focusing junction), where at a junction fluidly connected to a aqueous solution reservoir, a stream of said aqueous solution is introduced into a fluorinated oil comprising about 5% by weight (w/w) of a fluoro-surfactant, which results in the formation of a first set of water-in-oil droplets.

The process according to the present invention comprises the step of:
- replicating the plasmids using the polymerase capable of rolling circle replication of plasmids and concomitantly expressing the candidate peptides or pro-peptides encoded thereon using the in vitro transcription/translation system, within the water-in-oil droplets, to form droplets comprising each a candidate peptide, wherein if a pro-peptide is expressed, the pro-peptide is hydrolysed to form droplets comprising each a candidate peptide.

Any polymerase capable of rolling circle replication of plasmids may be used in the context of the present invention, and preferably is a polymerase with proof-reading activity. It is understood that the aqueous liquid should also comprise the needed reactants for the performance of the polymerase activity, such as dNTPs and suitable primers. As an example of a suitable polymerase, EquiPh i29^{™} DNA Polymerase, commercially available from Thermo Fisher Scientific may be used in conjunction with Exo-Resistant Random Primer, commercially available from Thermo Fisher Scientific. Ideally, the polymerase is allowed to replicate the plasmid for at least two hours, preferably at least three hours, or from three to twenty-four hours or from three to four hours. In a preferred embodiment, the polymerase capable of rolling circle replication of plasmids is a polymerase capable of rolling circle replication of plasmids at a temperature of 30°C to 45°C, and more preferably at a temperature of 30°C to 42°C. In a preferred embodiment, rolling circle replication of plasmids is achieved by pooling the water-in-oil droplets and placing them at a temperature of 30°C to 45°C for from three to twenty-four hours or from three to four hours, such as for example in a thermocycler.

The *in vitro* transcription/translation system used for the expression of the candidate peptides encoded on the plasmid may preferably be a cell-free *in vitro* transcription/translation system such as IVTT (*in vitro* transcription and translation) reagent available commercially from PUREfrex kit (GeneFrontier), a newly developed reconstituted cell-free protein synthesis reagent based on PURE system technology published by Professor Takuya Ueda in the University of Tokyo (Shimizu et al., 2001, Nat. Biotechnol., 19, 751. Ideally, the expression of the candidate peptides encoded on the plasmid is allowed to proceed for at least two hours, preferably at least three hours, or from three to twenty-four hours or from three to four hours. Accordingly, the *in vitro* transcription/translation system used for the expression of the candidate peptides encoded on the plasmid may preferably be an *in vitro* transcription/translation system derived from prokaryotes, such as for example from *E. coli.* In a preferred embodiment, expression of the candidate peptides encoded on the plasmid is achieved by pooling the water-in-oil droplets and placing them at a temperature of 30°C to 45°C for from three to twenty-four hours, or from three to four hours such as for example in a thermocycler.

The process according to the present invention comprises the step of:
- providing a second set of water-in-oil droplets, where each droplet comprises a one or more cells having a G protein-coupled receptor expressed on its surface, where said G protein-coupled receptor has a reporter moiety coupled to it and said reporter moiety is capable of emitting a signal, preferably an electromagnetic radiation signal, upon functional activation of the G protein-coupled receptor by a suitable candidate peptide.

In order to form and provide a second set of water-in-oil droplets where each droplet comprises at least a one or more cells having a G protein-coupled receptor expressed on its surface, said cells having a G protein-coupled receptor expressed on its surface are included in an aqueous liquid, such as for example an aqueous cell culture buffer, so that when the water-in-oil droplets are formed, a majority of the droplet comprises a single cell having a G protein-coupled receptor expressed on its surface and an amount of aqueous liquid. In order to ensure that essentially each formed droplet comprises at least a single cell, the concentration of cells in the aqueous liquid comprising the cells from which the droplets are formed must be chosen within a suitable range. Thus, the term "a majority of the droplet comprises at least a single cell having a G protein-coupled receptor expressed on its surface" should be understood as meaning that at least about 65%, preferably at least about 75% of the droplets comprise one cells, and the remainder of the droplets comprise two or more cells. An occupancy of one or two cells per droplet is acceptable, since it ultimately means that more sensors are available. In this way, the throughput may be further enhanced since either a droplet comprising one or two cells may subsequently be merged to a water-in-oil droplet comprising the candidate peptide.

The G protein-coupled receptor has a reporter moiety coupled to it that is capable of emitting a signal upon functional activation of the G protein-coupled receptor by a suitable candidate peptide. This allows to determine if binding of a suitable candidate peptide can lead to the functional activation the G protein-coupled receptor by detecting the signal using a suitable apparatus. In a preferred embodiment of the process according to the present invention, the G protein-coupled receptor (GPCR) reporter moiety is capable of emitting an optical signal, an in particular is capable of emitting a fluorescence signal when illuminated with a light source. As an example of a G protein-coupled receptor having a fluorescent reporter moiety coupled to it, the G protein-coupled receptors disclosed in EP 3 544 992 A4, whose sequences are herewith incorporated by reference.. It is understood that a reporter moiety capable of emitting a signal may as well be a reporter moiety capable of emitting a non-fluorescent signal, such as for example a reporter moiety capable of bringing about a change in the state of the cell having a G protein-coupled receptor expressed on its surface, such as for example expression of a reporter protein or a programmed cell death. While the inventors, in the context of the present invention, implemented a screening method using a G protein-coupled receptor (GPCR) reporter moiety that is capable of emitting an fluorescence signal when illuminated with a light source when binding a ligand, it is understood that the present invention is not limited to a reporter moiety that is capable of emitting an fluorescence signal when illuminated with a light source but can also encompass a reporter moiety that is capable of quenching an fluorescence signal upon binding a ligand. Thus, the G protein-coupled receptor (GPCR) reporter moiety that is capable of emitting an optical signal should be understood as meaning a reporter moiety that is capable of an increase or a decrease of an optical signal upon binding a ligand.

The process according to the present invention comprises the step of:
- merging the droplets comprising a candidate peptide from the first set with the droplets comprising a one or more cells having a G protein-coupled receptor expressed on its surface from the second set, in a one-to-one fashion, to form a third set of combined droplets.

Merging each one of the droplets comprising a candidate peptide with one droplet comprising a one or more cells having a G protein-coupled receptor expressed on its surface, in a one-to-one fashion, to form a third set of combined droplets, may be carried out in a microfluidic device. For instance, merging may be brought about by exposing the two droplets to be merged to an electrical field pulse provided by electrodes when they are brought into proximity of each other in the microfluidic device.

In a preferred embodiment of the process according to the present invention, the step of merging each one of the droplets comprising a candidate peptide with one droplet comprising a one or more cells having a G protein-coupled receptor expressed on its surface, in a one-to-one fashion, to form a third set of combined droplets is preferably temperature-controlled, and more preferably is carried out at about 4°C to 20°C and most preferably at about 4°C. At 4°C, the metabolic rate of the single cell having a G protein-coupled receptor expressed on its surface comprised in the droplet is slowed, which avoids a decline in viability after less than one hour that is otherwise observed when the step is carried out at temperatures of about 20°C or more.

A suitable microfluidic device suitable for droplet merging may comprise a flow-focusing junction (e.g. 50 µm width) for single-cell encapsulation, a droplet following junction (e.g. 20 µm width), a droplet merging structure (e.g. including a pressurized area with 40 µm width and a decompressive area with 60 µm width) and electrode channels (e.g. 100 µm width). The electrode channels can be filled with 5 M NaCl salt solution to form the electrode. The merging of droplets can be triggered in an electric field which may be applied by electrodes connected to a high-voltage amplifier and a power supplier with a voltage output of 500 V at a frequency of 1 kHz. General pressure used for oil, cell suspension and the droplets within the microfluidic device is about 800 mbar, 700 mbar and 850 mbar, respectively.

The process according to the present invention comprises the step of:
- in the third set of combined droplets, separating the combined droplets that emit a signal above a predetermined signal intensity threshold, to form a fourth set of combined droplets, from the combined droplets that emit a signal below a predetermined signal intensity threshold.

Probing the combined droplet for a signal emitted by the reporter moiety will depend on the specific type of the signal emitted by the reporter moiety. For instance, when the reporter moiety is capable of emitting an optical signal, such as for example a fluorescence signal, the probing may be carried out by exposing the combined droplet to a radiation having a first wavelength, emitted by a radiation source of the microfluidic device, and then detecting a radiation of a different, second wavelength that is emitted by the fluorescent reporter moiety, via a radiation sensor of the microfluidic device. Such radiation having a first wavelength may be chosen from VIS, UV or IR radiation, for example. Probing the combined droplet for a signal emitted by the reporter moiety via a first radiation that elicits a fluorescence signal from the fluorescent reporter moiety has the advantage of allowing a high throughput of combined droplet, since the response of the fluorescent reporter moiety to a radiation having a first wavelength is practically instantaneous. In particular, when the signal moiety is activated by a conformational shift due to the binding of the candidate peptide ligand, there is no lag before the combined droplets can be probed. Nonetheless, when the reporter moiety is capable of emitting a non-optical signal, such as for example a such as for example expression of a reporter protein or a programmed cell death, the combined droplet may also be probed via a suitable probing protocol. For example, dyes that stain dead cells that may be detected via fluorescence are well-known. Also, reporter protein that may be detected via fluorescence are also well-known.

In any case, a predetermined signal intensity threshold is defined to ensure that the peptides that functionally activate the G protein-coupled receptor expressed on the surface of the cells are identified. For example, the predetermined signal intensity threshold may be defined as a signal intensity that is above the signal intensity observed for a wildtype agonist peptide ligand of a particular G protein-coupled receptor for which alternative agonist peptide ligands are being investigated.

When the reporter moiety is capable of emitting an optical signal, such as for example a fluorescence signal, the probing may be carried out by exposing the combined droplet to a radiation having a first wavelength and then detecting a radiation of a different, second wavelength that is emitted by the fluorescent reporter moiety. Suitable devices for probing the combined droplets comprise a radiation source emitting a radiation having a first wavelength and a radiation detector detecting radiation of a second wavelength that is emitted by the fluorescent reporter moiety. Such devices are for example, cell sorting devices such as fluorescence-activated cell sorting devices (FACS).

Separating the combined droplets that emit a signal above the predetermined signal intensity threshold from the third set of combined droplets may be carried out via several methods, such as for example by deflection of the droplets that emit a signal above the predetermined signal intensity threshold passing an electrical field, which electrical field is preferably orthogonal to the direction of the passage of the droplets. While the droplet may for example be deflected while falling vertically through the electrical filed generated by two electrodes, as in fluorescence-activated cell sorting devices (FACS), the combined droplets that emit a signal above the predetermined signal intensity threshold are preferably separated by passing an electrical field that extends across a microfluidic channel of a microfluidic device through which the combined droplets flow. In the latter case, the droplets move with the flow of the water-in-oil emulsion comprising the droplets. The combined droplets that do not emit a signal above the predetermined signal intensity threshold may also the separated from the droplets, using the same procedure as above. For instance, where the electrical field deflects the cells in the microfluidic device, a Y-junction may be used immediately downstream of the electrical field.

It is understood that the separation of the combined droplet may result in all "positive" combined droplets being pooled into a single receptacle when the combined droplets are deflected into the same receptacle, or may result in each of the "positive" combined droplets being individually placed in a separate receptacle when the combined droplets are each deflected into a separate receptacle.

The process according to the present invention comprises the step of:
- in the fourth set of combined droplets, determining the sequences of the peptides or pro-peptides encoded on the plasmids.

After separation, the plasmids from the combined droplets that emit a signal above the predetermined signal intensity threshold are isolated and may be further amplified in a dedicated polymerase chain reaction, which may for example be achieved by rolling circle amplification of the entire plasmid or may be amplified by amplification of the plasmid DNA in the presence of primers that are designed to selectively amplify the sequence encoding the candidate peptide. In any case, the amplified plasmid or fragment may then be cloned into an expression vector to produce the candidate peptide for further study of it effect on the functional activation of the GPCR under study and/or may be sequenced to determine the coding sequence of the candidate peptide or pro-peptide.

Determining the sequences encoding the candidate peptides in said plasmids may be achieved via known sequencing methods in the art, such as for example Sanger sequencing, NGS sequencing, nanopore sequencing, pyrosequencing, and others. When all "positive" combined droplets pooled into a single receptacle are sequenced in bulk, the sequences encoding the candidate peptides in said plasmids will be consensus sequences reflecting conserved and variable positions in the sequences encoding the candidate peptides. When the "positive" combined droplets are individually placed in an separate receptacle, the sequence encoding the candidate peptides clone may be sequenced individually, such as to provide a sequence that unambiguously encodes the candidate peptide.

In one preferred embodiment of the first object according to the present invention, the G protein-coupled receptor is a G protein-coupled receptor comprising a fluorescent reporter moiety, and in particular where the reporter moiety is an intracellular reporter moiety. The fluorescent reporter moiety may be a fluorescent reporter moiety that is integrated into the third intracellular loop of the G protein-coupled receptor, analogously to the G protein-coupled receptors reported in US20210101960A1.

In one preferred embodiment of the first object according to the present invention, the cell is a human cell, such as HEK cells. An exemplary HEK cell line is HEK 293T.

In one preferred embodiment of the first object according to the present invention, wherein probing the combined droplet for a signal emitted by the reporter moiety and separating the combined droplets that emit a signal above the predetermined signal intensity threshold is carried out via fluorescence-activated droplet sorting (FADS). Fluorescence-activated droplet sorting (FADS) is a technology used to sort and droplets that encapsulate individual cells or particles based on the fluorescent properties of the droplets or their content. FADS is not a patented technology. Fluorescence-activated droplet sorting (FADS) is described in Baret et al. Lab Chip, 2009, 9, 1850.

In one preferred embodiment of the first object according to the present invention, the plasmids from the combined droplets that emit a signal above the predetermined signal intensity threshold, after separation, are pooled and the sequences encoding the candidate peptides in said plasmids determined as consensus sequences. Stated alternatively, the combined droplets that emit a signal above the predetermined signal intensity threshold are pooled together in one receptacle and are sequenced in bulk, which yields a sequence that will exhibit conserved and variable portions.

In one preferred embodiment of the first object according to the present invention, the plasmids from the combined droplets that emit a signal above the predetermined signal intensity threshold are isolated individually and the sequences encoding the candidate peptides in said plasmids determined individually. Stated alternatively, each plasmid is sequenced individually and thus each sequence obtained can unambiguously be attributed to the candidate peptide of the respective combined droplet that led to a signal above the predetermined signal intensity threshold.

In one preferred embodiment of the first object according to the present invention, the formed water-in-oil droplets further comprise a proteinase capable of hydrolysing the pro-peptide of the candidate peptide and releasing the candidate peptide. When expressing candidate peptides in the droplets, the peptides may be expressed first as pro-peptides that are subsequently hydrolysed to yield the candidate peptide proper. For instance, the pro-peptide may comprise a target sequence, in general on the N-terminal side, or an affinity tag sequence that are best removed from the pro-peptide if the interaction between the candidate peptide and the G protein-coupled receptor is to be free from interference by the target sequence or affinity tag sequence and identical to the native interaction.

In one preferred embodiment of the first object according to the present invention, the pro-peptide of the candidate peptide is a fusion peptide having a tobacco etch virus (TEV) protease cleavage site on its n-terminal side.

In one preferred embodiment of the first object according to the present invention, the formed water-in-oil droplets further comprise a tobacco etch virus protease capable of hydrolysing the pro-peptide of the candidate peptide, such as for example a fusion protein, and releasing the candidate peptide. Tobacco etch virus protease exhibits a high sequence specificity, and is thus suitable for the controlled hydrolysis of fusion proteins, such as for example to remove an N-terminal affinity tag from a fusion peptide, provided the peptide sequence is included at the correct location in the peptide and is the only peptide sequence that is accessible to TEV protease.

In one preferred embodiment of the first object according to the present invention, the diameter of the water droplets is between 20 to 25 µm. The diameter of the water droplets may be influenced during the forming of said droplets, by varying the flow rate of the aqueous phase and/or oil phase.

In one preferred embodiment of the first object according to the present invention, the reporter moiety is capable of emitting an optical signal above the predefined threshold value within 0.001 to 1 s, preferably within 0.001 to 0.1 s, of functional activation of the G protein-coupled receptor by a suitable candidate peptide, which allows probing within that time. This is considerably faster than a reporter moiety capable of emitting a non-optical signal such as programmed cell death or expression of a reported protein, and this avoids a lag time after merging the droplets, which may then immediately be probed for a signal and sorted.

In one preferred embodiment of the first object according to the present invention, the G protein-coupled receptor is PACLight1, an engineered G protein-coupled receptor according to sequence SEQ ID1, which is capable of emitting a fluorescence signal upon functional activation with a ligand.

### EXAMPLES

To identify peptide agonists that functionally activate a G protein-coupled receptor expressed in a cell, a process for screening a complete plasmid library encoding for peptides according to the present invention was carried out. The G protein-coupled receptor in this case was PACLight1, expressed in HEK cells. PACLight1 has a sequence according to SEQ ID1.

The plasmid library encoded for random penta-peptides and thus provided for a library size of about 3 million (20⁵) peptides.

The process incorporated rolling circle amplification (RCA) of single plasmids, *in vitro* transcription and translation (IVTT), and peptide cutting using Tobacco Etch Virus protease into a simplified process.

In a first step, an aqueous liquid comprising, among others, the plasmid DNA was provided, from which oil-in-water droplets were formed in a continuous phase of fluorinated oil (HFE-7500) in a microfluidic device. The concentration of DNA was adjusted to 100 fg/µL, a concentration at which, as per the Poisson distribution, an anticipated 10% single-cell occupancy and 2% doublet occupancy within the droplets is achieved. The aqueous liquid further comprised a suitable concentration of the RCA polymerase and of the IVTT for replication and transcription of the plasmid DNA in the droplet as well as a suitable concentration of a TEV protease to allow the proteolysis of the pre-peptide and the release of the penta-peptides under investigation.

In order to arrive at a functional droplet assay, the previously produced droplets comprising the expressed penta-peptide were merged with droplets comprising the G protein-coupled receptor PACLight1, expressed in HEK cells, in a microfluidic using a droplet merging device.

The droplet merging device was fabricated by PDMS with 35 µm height, containing a flow-focusing junction (50 µm width) for single-cell encapsulation, a droplet synchronization region (20 µm width) and droplet merging part (including a pressurized area with 40 µm width and a decompressive area with 60 µm width) and electrode channels (100 µm width).

Electrodes were made by filling the channels with 5 M NaCl salt solution. The droplet merging happened at a pressurized and decompressive structure in the main channel. Droplet merging was triggered by an electric field which was applied by electrodes connected to a high-voltage amplifier and a power supplier with a voltage output of 500 V at a frequency of 1 kHz. General pressure used for oil, cell suspension and the droplets after the incubation were 800 mbar, 700 mbar and 850 mbar, respectively, resulting in the droplets after the reaction being diluted by around 10-fold.

A fluorescence intensity of 1.0, equal to the fluorescence signal resulting from the incubation of the antagonist PACAP ₆₋₃₈ with a cell expressing PACLight1 was set to probe, identify, and subsequently separate the combined droplets that emitted a signal above this signal intensity via fluorescence-activated droplet sorting (FADS) device.

PACAP₆₋₃₈ has the sequence according to SEQ ID2. PACAP stands for Pituitary Adenylate Cyclase Activating Polypeptide.

PACAP ₁₋₃₈ has the sequence according to SEQ ID3. PACAP stands for Pituitary Adenylate Cyclase Activating Polypeptide.

In total, 25 candidates were identified and separated from the library based on the above criteria. For each candidate, the plasmid from the combined droplets that emitted a signal above the predetermined signal intensity threshold were isolated and the sequences encoding the candidate peptides in said plasmids were determined via Sanger sequencing. Before Sanger sequencing, the plasmid DNA was amplified via Q5^{®} High-Fidelity DNA Polymerase, purified by DNA electrophoresis, gel extraction, and PCR clean-up.

To confirm the efficacy of the selected 25 peptides, the sequences encoding the candidate peptides were expressed and incubated with cells expressing the PACLight1 receptor, in a separate experiment, and it was observed that 100% of the thus identified peptide candidates effectively functionally activated the G-protein receptor such that the recorded fluorescence was similar to that recorded for the agonist PACAP ₁₋₃₈. For each penta-peptide, 100 fg/µL of DNA encoding the respective penta-peptide was used in combination with the IVTT system to express the penta-peptide.

The peptide sequences of the twenty-five penta-peptides are shown in Fig. 1A and 1B, as well as the recorded relative mean fluorescence of the merged droplets.

Thus, the screening process according to the present invention is able to quickly and efficiently isolate penta-peptides that can functionally activate a particular G-protein coupled receptor from a vast library of penta-peptides. It is understood, that in principle, the plasmids can be engineered to represent an even larger library of possible ligands for any type of receptors that can produce a detectable signal upon interacting with the ligands. Stated alternatively, the present invention can be applied to any ligand-receptor pair in animal cells, provided the receptor is expressed on the surface of the animal cell and includes a reporter moiety that is capable of emitting a signal upon functional activation of the receptor.

### LIST OF REFERENCE SIGNS

none

## Claims

1. A process for screening a plasmid library encoding for peptides for one or more peptides that functionally activate a G protein-coupled receptor in a cell, comprising the step of:
- providing a first set of water-in-oil droplets comprising each a single plasmid encoding a candidate peptide or a pro-peptide thereof, a polymerase capable of rolling circle replication of plasmids, and an *in vitro* transcription/translation system, and optionally a protease,
- replicating the plasmids using the polymerase capable of rolling circle replication of plasmids and concomitantly expressing the candidate peptides or pro-peptides encoded thereon using the *in vitro* transcription/translation system, within the water-in-oil droplets, to form droplets comprising each a candidate peptide, wherein if a pro-peptide is expressed, the pro-peptide is hydrolysed to form droplets comprising each a candidate peptide,
- providing a second set of water-in-oil droplets, where each droplet comprises a one or more cells having a G protein-coupled receptor expressed on its surface, where said G protein-coupled receptor has a reporter moiety coupled to it and said reporter moiety is capable of emitting a signal, preferably an electromagnetic radiation signal, upon functional activation of the G protein-coupled receptor by a suitable candidate peptide,
- merging the droplets comprising a candidate peptide from the first set with the droplets comprising a one or more cells having a G protein-coupled receptor expressed on its surface from the second set, in a one-to-one fashion, to form a third set of combined droplets,
- in the third set of combined droplets, separating the combined droplets that emit a signal above a predetermined signal intensity threshold, to form a fourth set of combined droplets, from the combined droplets that emit a signal below a predetermined signal intensity threshold,
- in the fourth set of combined droplets, determining the sequences of the peptides or pro-peptides encoded on the plasmids.

2. The process for screening a plasmid library according to claim 1, wherein G protein-coupled receptor is a G protein-coupled receptor comprising a fluorescent reporter moiety, and in particular where the reporter moiety is an intracellular fluorescent reporter moiety, such as green fluorescent protein.

3. The process for screening a plasmid library according to claim 1 or 2, wherein the cells having a G protein-coupled receptor expressed on its surface are mammalian cells, preferably human cells such as HEK cells.

4. The process for screening a plasmid library according to any one claim 1 to 3, wherein separating the combined droplets that emit a signal above the predetermined signal intensity threshold is carried out via fluorescence-activated droplet sorting (FADS).

5. The process for screening a plasmid library according to any one claim 1 to 4, wherein the sequences of the peptides or pro-peptides encoded on the plasmids are determined by pooling the combined droplets that emit a signal above the predetermined signal intensity threshold and the sequences encoding the candidate peptides in said plasmids are determined as consensus sequences.

6. The process for screening a plasmid library according to any one claim 1 to 4, wherein the sequences of the peptides or pro-peptides encoded on the plasmids are determined by individually isolating the combined droplets that emit a signal above the predetermined signal intensity threshold and the sequences encoding the candidate peptides in said plasmids are determined individually.

7. The process for screening a plasmid library according to any one claim 1 to 6, wherein the first set of water-in-oil droplets comprise a protease capable of hydrolysing the pro-peptide of the candidate peptide and releasing the candidate peptide.

8. The process for screening a plasmid library according to claim 7, wherein the proteinase capable of hydrolysing the pro-peptide of the candidate peptide and releasing the candidate peptide.

9. The process for screening a plasmid library according to any one claim 1 to 8, wherein the diameter of the water droplets is between 20 to 25 µm.

10. The process for screening a plasmid library according to any one claim 1 to 9, wherein the reporter moiety is capable of emitting an electromagnetic radiation signal, in particular a fluorescence signal, within 0.001 to 1 s, preferably within 0.001 to 0.1 s, of functional activation of the G protein-coupled receptor by a suitable candidate peptide.

11. The process for screening a plasmid library according to any one claim 1 to 10, wherein the G protein-coupled receptor is based on human PAC1 receptor (hPAC1 R), and preferably is PACLight1 having a sequence according to SEQ ID1.

12. A microfluidic device configured to carry out the process according to any one claim 1 to 11, comprising
- a microfluidic formation chamber for forming a first set of water-in-oil droplets, each comprising a single plasmid encoding a candidate peptide or a pro-peptide thereof, a polymerase capable of rolling circle replication of plasmids, and an in vitro transcription/translation system, and optionally a protease,
wherein the formation chamber is fluidly connected to a reservoir of oil and a reservoir of an aqueous liquid comprising plasmids of a plasmid library, each plasmid comprising a sequence encoding a candidate peptide or a pro-peptide thereof, and a polymerase capable of rolling circle replication of plasmids, and an in vitro transcription/translation system, and optionally a protease,
- a microfluidic merging chamber fluidly connected to the formation chamber, for receiving the water-in-oil droplets from the formation chamber, for merging droplets of the first set comprising a candidate peptide with droplets of a second set, said droplets comprising a one or more cells having a G protein-coupled receptor expressed on its surface, in a one-to-one fashion, to form a third set of combined droplets,
wherein the merging chamber preferably comprises electrodes capable of generating an electrical field across the merging chamber,
- a sorting chamber fluidly connected to the merging chamber, for receiving the combined droplets and separating the combined droplets that emit a signal above a predetermined signal intensity threshold, to form a fourth set of droplets, from the combined droplets that emit a signal below a predetermined signal intensity threshold,
- and optionally a collection chamber, for receiving the separated combined droplets that emit a signal above a predetermined signal intensity threshold.
